# EUROPEAN PATENT APPLICATION

(11) **EP 1 182 205 A1**
(43) Date of publication of application: **27.02.2002**
(21) Application number: 00118437.3
(22) Date of filing: 24.08.2000
(51) Int. Cl.: C07F 9/50, C07F 9/655, C07C 29/09, C07B 53/00, C07F 15/00, C12P 41/00, C12P 7/18, B01J 31/24

(54) **Chiral diphosphines via 2,3 -sigmatropic rearrangement.Application in asymmetric transition metal catalysis**

(71) Applicant: PPG-Sipsy S.C.A., 49242 Avrille Cedex (FR)
(72) Inventor: Demay, Stéphane, 91160 Saulx-Les-Chartreux (FR); Kotschy, Andras, 1031 Budapest (HU); Knochel, Paul, 81475 München (DE); Volant, Florence, 29100 Douarnennez (FR); Lotz, Matthias, 81375 München (DE)
(74) Representative: Sternagel, Fleischer, Godemeyer & Partner Patentanwälte

(57) **Abstract**

The present invention refers to Chiral diphosphines selected from
a) diphosphines corresponding to the formula 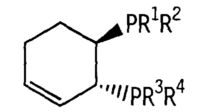 wherein R¹ - R⁴ are each independently selected from substituted and unsubstituted hydrocarbyl groups,
b) diphosphines corresponding to the formula 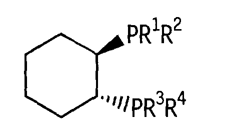 wherein R¹ - R⁴ are each independently selected from substituted and unsubstituted hydrocarbyl groups with the proviso that if R¹ and R² is phenyl at least one of R³ and R⁴ is different from phenyl
c) diphosphines corresponding to the formula 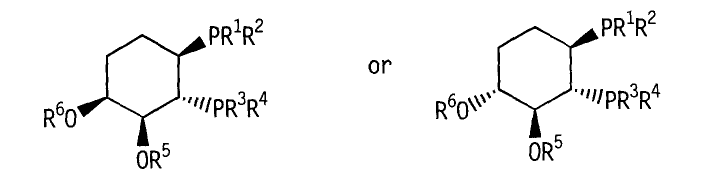 wherein R¹ - R⁴ are each independently selected from substituted and unsubstituted hydrocarbyl groups and R⁵ and R⁶ are each independently selected from the group consisting of hydrogen, alkyl and residues of protecting groups, whereby R⁶ and R⁵ taking together may be an divalent substituent thereby forming a bicyclus, to a method of making same, to a method for preparing a chiral precursor suitable for making the new chiral diphosphines, to transition metal complexes comprising the chiral diphosphines of the present invention, and to the use of either the chiral diphosphines or their transition metal complexes in asymmetric synthesis.

## Description

The present invention refers to new chiral diphosphines, to a method of making same, to a method for preparing a chiral precursor suitable for making the new chiral diphosphines, to transition metal complexes comprising the chiral diphosphines of the present invention, and to the use of either the chiral diphosphines or their transition metal complexes in asymmetric synthesis.

Chiral 1,2-diphosphines are important ligands for asymmetric metal catalysis. A survey of suitable systems is given in Noyori, R. Asymmetric Catalysis in Organic Synthesis; Wiley Interscience, 1994.

In Paul Knochel et al. Tetrahedron Letters 40 (1999)4981-4984, the stereoselective preparation of phosphine oxides via a 2,3-sigmatropic shift of allylic diphenylphosphinites is disclosed. Starting from the chiral (1R,2R) trans-cyclohex-3-en-1,2-diol due to the stereoselectivity of the [2,3]-sigmatropic rearrangement the chiral trans-1,2-bis(diphenylphosphinooxide)cyclohex-3-en can be prepared. But this chiral phosphine oxide compound does not show activity in asymmetric synthesis.

In EP-A-117 156 a process for the preparation of trans-1,2-(dihalophosphino)cycloalkanes is described. Thereby C₃- to C₁₂-cycloalkines are reacted with a trivalent phosphorus compound, preferably phosphorus trichloride in presence of elemental phosphorus. These intermediates are transformed into the hydrocarbylphosphino cycloalkanes by reaction with a suitable carbon nucleophile, for instance a suitable Grignard reagent. As exemplified with trans-1,2-bis(diphenylphosphino)cyclopentane the reaction product is racemic and separation of the enantiomers is achieved by forming and crystallizing the corresponding nickel (II) halide complex. The respective enantiomers are separated manually by crystal picking and then the enantiomer of the respective bis(phosphino)cyclopentane is recovered by treatment with sodium cyanide. This reference thus discloses a method for preparation of chiral trans-1,2-bis(diphenylphosphino)cyclopentane that is not industrially applicable and it can also not be expected that other chiral (diphospino)cycloalkanes can be obtained by this method.

EP-A-551 642 refers to the diastereoselective hydrogenation of folic acid in presence of a rhodium complex of an optically active diphosphine. Among other chiral diphosphines (S,S)-(+)-1,2-bis(diphenylphosphino)cyclohexane is disclosed. Use of the corresponding rhodium complex does lead to a negligible excess of one of the diastereomers. Thus the activity of chiral 1,2-bis(diphenylphosphino)cyclohexane in asymmetric metal catalysis could not be proven in EP-A-551 642.

Axel Kleemann et al. in Synthesis (1981) 740 discloses the asymmetric hydrogenation of dehydrodipeptides using complexes of rhodium with different chiral phosphino ligands, inter alia (R,R)-1,2-bis(diphenylphosphino)cyclohexane. The optical yields achieved in the asymmetric hydrogenation discussed in these references with the above rhodium catalyst are still very low.

Thus the object of the present invention is to provide a versatile route to chiral disphosphine ligands that induce high activity in asymmetric synthesis.

This object has been attained by chiral diphosphines selected from
a) diphosphines corresponding to the formula 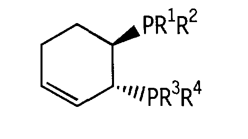 wherein R¹ - R⁴ are each independently selected from substituted and unsubstituted hydrocarbyl groups,
b) diphosphines corresponding to the formula 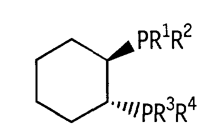 wherein R¹ - R⁴ are each independently selected from substituted and unsubstituted hydrocarbyl groups with the proviso that if R¹ and R² is phenyl at least one of R³ and R⁴ is different from phenyl
c) diphosphines corresponding to the formula 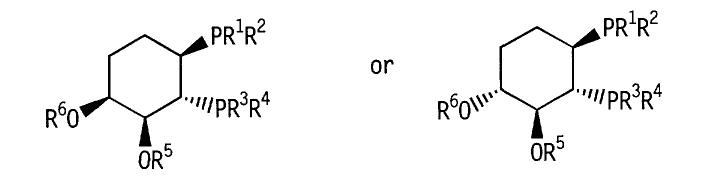 wherein R¹ - R⁴ are each independently selected from substituted and unsubstituted hydrocarbyl groups and R⁵ and R⁶ are each independently selected from the group consisting of hydrogen, alkyl and residues of protecting groups, whereby R⁶ and R⁵ taking together may be an divalent substituent thereby forming a bicyclus.

The chiral diphosphines according to group a) of the present invention can be prepared by reacting a chiral 1,2-diphosphine oxide corresponding to the formula 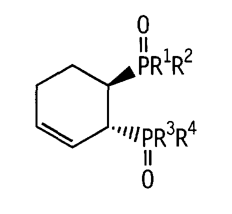 with a stoichiometric excess of hydrosilane, wherein R¹ - R⁴ are each independently selected from substituted and unsubstituted hydrocarbyl groups. Preferred hydrosilanes are trichlorosilane, triethylsilane and diphenylsilane, trichlorosilane being most preferred. Preferably this reaction is carried out in an organic solvent such as toluene in an autoclave under inert atmosphere and elevated temperature and pressure.

According to one preferred embodiment the starting chiral 1,2-diphospine oxide of the above defined process is prepared by first reacting the chiral (1R,2R) trans-cyclohex-3-en-1,2-diol with ClPR¹R² wherein R¹ and R² are each independently selected from unsubstituted hydrocarbyl groups or substituted hydrocarbyl groups to obtain the corresponding diphosphinite and thereafter effecting a [2,3]-sigmatropic rearrangement by heating the diphosphinite to obtain the chiral 1,2-diphosphine oxide. Reaction with an excess of hydrosilane as described above then yields a chiral diphosphine having identical phosphino groups.

Alternatively the starting chiral (1R,2R) trans-cyclohex-3-en-1,2-diol may be reacted with a compound to protect one hydroxy functionality. Silyl protecting groups are especially preferred because they can be removed in mild conditions compatible with the presence of sensitive phosphinites. Then the resulting product is reacted with ClPR¹R² to obtain the corresponding monophosphinite. Thereafter the protecting group is removed and the resulting hydroxyfunctional compound is reacted with ClPR³R⁴. R¹ - R⁴ are again each independently selected from substituted and unsubstituted hydrocarbyl groups, with the proviso that at least one of R¹ and R² is different from R³ and R⁴. The resulting diphosphinite is thereafter heated to effect a [2,3]-sigmatropic rearrangement to obtain the chiral 1,2-diphosphine oxide having different diphosphino oxide groups while maintaining the chirality of the compound.

An alternative route to the mixed 1,2-diphosphine oxides is to first react the starting chiral (1R,2R) trans-cyclohex-3-en-1,2-diol with a compound to protect one hydroxy functionality followed by reacting the unprotected hydroxy group with ClPR¹R² to yield the corresponding monophosphinite. This compound is then refluxed in an organic solvent, preferably toluene or mesitylene in order to obtain the 1-phosphine-oxide, 4-ether-cyclohex-2-en. Thereafter the protecting group is removed and the hydroxy functionality is reacted with ClPR³R⁴ to yield the corresponding phosphinite compound. After refluxing said phosphinite compound the desired chiral 1,2-diphosphine oxide having different diphosphino oxide groups can be isolated. As above R¹ - R⁴ are each independently selected from substituted and unsubstituted hydrocarbyl groups, with the proviso that at least one of R¹ and R² is different from R³ and R⁴

This method is further illustrated by following reaction scheme, wherein Prot is a protecting group:

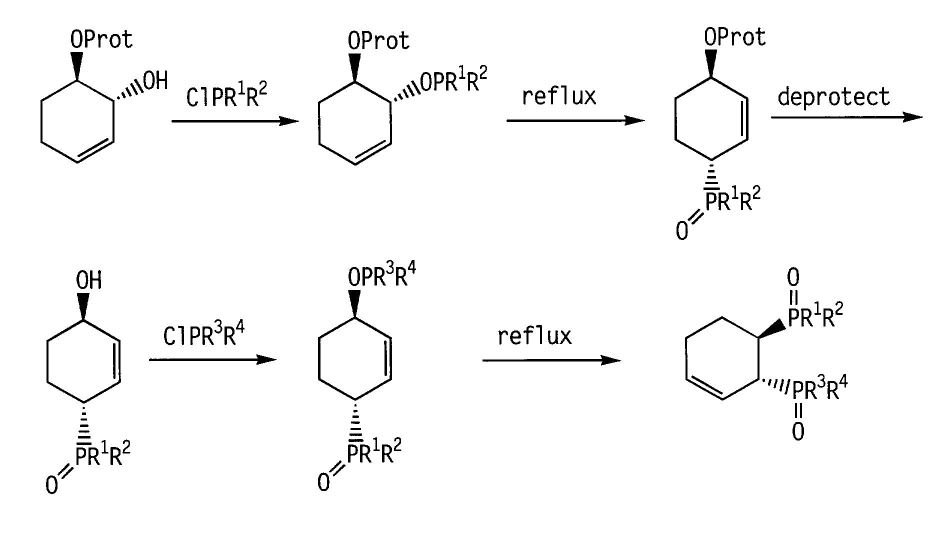

Suitable substituents for R¹ - R⁴ as used herein through out the entire specification are alkyl, preferably C₁- to C₁₀-alkyl, cycloalkyl, preferably C₅- to C₁₀-cycloalkyl, aryl, preferably phenyl, alkaryl and aralkyl. These substituents may be substituted with hydrocarbyl groups as defined above, or with substituents bearing heteroatoms such as halogen, ether groups, amino groups, amide groups and cyano groups.

The above [2,3]-sigmatropic rearrangement is preferably conducted by dissolving the corresponding diphosphinite in an organic solvent, preferably an aromatic solvent such as toluene or mesitylene and refluxing the solution for a time sufficient to complete the reaction.

An important advantage of the method of the present invention is that the substituents on the phosphino oxide groups can be easily varied in order to yield differently substituted chiral diphosphines. Thereby chiral diphosphines that are tailored to fulfill different requirements can be readily synthesized by variation of the different substituents R¹ - R⁴. Besides the possibility to introduce a vast number of different substituents on the phosphorus atoms of the chiral 1,2-diphosphine oxide according to the process of the present invention the diphosphine oxide can be easily reduced to the corresponding diphosphine by use of a stoichiometric excess of hydrosilane preferably trichlorosilane without affecting the double bond in the cyclohexene ring. Due to the presence of an olefinic functionality in the chiral diphosphines according to the present invention these compounds cannot only be used as ligand in the asymmetric synthesis but can be further derivatized.

Suitable derivatives are selected from
b) diphosphines corresponding to the formula 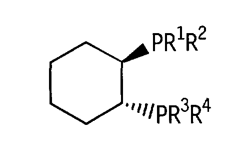 wherein R¹ - R⁴ are each independently selected from substituted and unsubstituted hydrocarbyl groups with the proviso that if R¹ and R² is phenyl one of R³ and R⁴ is different from phenyl;
c) diphosphines corresponding to the formula 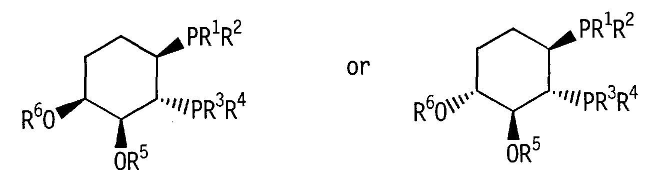 wherein R¹ - R⁴ are defined as above and R⁵ and R⁶ are each independently selected from the group consisting of hydrogen, alkyl and residues of protecting groups, whereby R⁶ and R⁵ taking together may be an divalent substituent thereby forming a bicyclus.

Suitable protecting groups may lead to acetals, ethers, silyl ethers, esters, carbamates, carbonates, thiocarbonates, thiocarbamates and thioesters.

These derivatives can be either prepared by a hydrogenation of the C-C-double bond of the chiral diphosphines according to the present invention or by cis-hydroxylation or trans-hydroxylation of the double bond and optional subsequent alkylation or protection of the resultant hydroxy functionalities. The cis-dihydroxylation is preferably carried out by catalytic osmylation whereas the trans-dihydroxilation is preferably made by epoxidation/hydrolysis, preferably with peracetic acid in presence of perchloric acid. Prior to derivatisation of the double bond the phosphino groups are preferably protected using a borane preferably BH₃ to avoid any reactions on the phosphino groups. Deprotection after derivatisation can be performed using a large excess of an amine preferably diethylamine. Alternatively, all these derivatisation reactions can be equally performed on the corresponding diphosphine oxides. These diphosphine oxides can be reduced in the same way as previously described using hydrosilane in excess to yield the above derivatives

The chiral (1R,2R) trans-cyclohex-3-en-1,2-diol that is used as starting compound according to the preferred method to make the chiral diphosphine compounds of the present invention can be made by sequence of the following steps:
1) reduction of 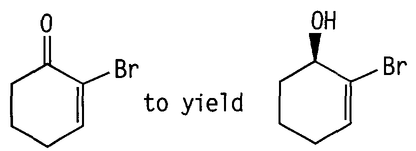
2) debromination and protection of the hydroxy functionality of 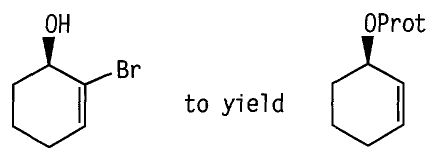 wherein Prot is a protecting group,
3) epoxidation of 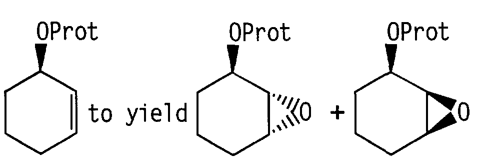
4) seperation of 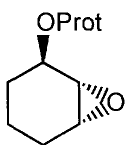
5) rearrangement of 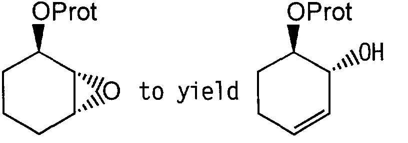
6) deprotection of

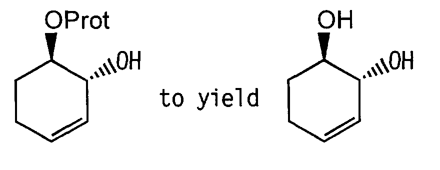

The reduction of 2-bromocyclohexenone is preferably conducted according to the CBS-reduction as disclosed in Corey, E.J.; Helal, C.J.; Angew. Chem . Int. Ed.; 1998; 37; 1986, using Me-CBS catalyst and Me₂SBH₃ in THF. Debromination is preferably achieved by treatment with t-BuLi, the protecting group is preferably introduced by silylation with TBDPSCl (tert.-butyldiphenylsilyl chloride). The resulting silylated alcohol is epoxidized by preferably using MCPBA (meta-chloroperbenzoic acid). This results in two diastereoisomeric epoxides whereby the major epoxide can be easily separated, for example by chromatography. Rearrangement of the major epoxide compound to yield the corresponding allylic alcohol is preferably conducted by treatment with Et₂NLi. The protection group is preferably removed by TBAF (tetrabutylammonium fluoride) to yield the desired chiral diol.

Alternatively the chiral trans-cyclohex-3-en-1,2-diol can be prepared starting from a racemic 3,4-trans-diacyloxy-cyclohexen, preferably 3,4-trans-diacetoxy-cyclohexen using an enzymatic process. Reaction for example in presence of *Pseudomas Fluorescens Lipase* results in only conversion of the (R,R) enantiomer of the starting compound as exemplified by 3,4-trans-diacetoxy-cyclohexen to the mono-hydroxy compound according to the following reaction scheme:

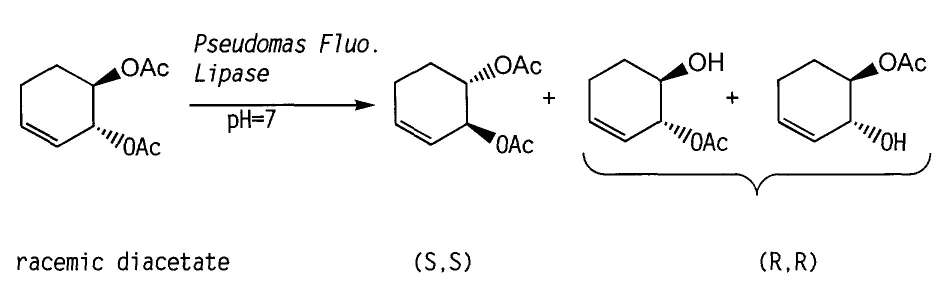

The diacetate can be separated from the mono-hydroxy compound and both fractions can be separately converted to the corresponding chiral diols. This method has the advantage that the desired chiral starting compound for the processes of the present invention can be made with considerably lesser steps and in higher yields compared to the first alternative. Additional both enantiomers can be obtained opening the possibility to make also both enantiomers of the chiral diphosphines of the present invention. Accordingly dependent on the enantiomer employed in the asymmetric syntheses both isomers of the product can be obtained. This possibility dramatically increases the usefulness of the present invention.

The following examples are provided to illustrate the present invention but are not intended limit the present invention.

### Example 1, Preparation of (1R,2R) trans-cyclohex-3-en-1,2-diol (1R,2R) trans-cyclohex-3-en-1,2-diol is prepared in accordance with the following reaction scheme:

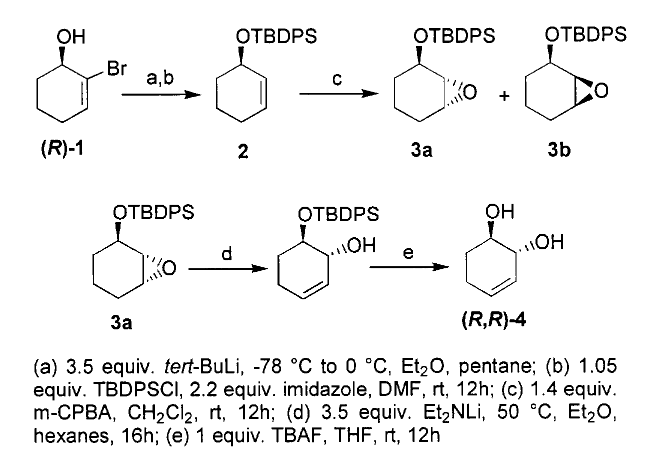

CBS-reduction of 2-bromocyclohexenone furnishes the allylic alcohol 1 in 95 % yield and 96-98 % *ee* using Me-CBS catalyst (15 mol %) and Me₂S.BH₃ (60 mol %) in THF at -15 °C. The debromination of 1 is readily achieved by treatment with *tert*-BuLi (3.5 equiv.) in pentane-ether at -78° C affording after the silylation with TBDPSC1 (1.05 equiv.) and imidazole (2.2 equiv.) in DMF, (rt, 18 h) the silylated alcohol 2. Epoxidation of 2 with MCPBA (1.4 equiv.) in CH₂Cl₂ (rt, 12 h) furnishes two diastereoisomeric epoxides 3a and 3b in the ratio 7:3. The major epoxide 3a is readily separated by chromatography (eluent pentane-ether) and isolated in 50 % yield from 1. Finally, 3a is submitted to a rearrangement by treatment with Et₂NLi in hexanes-ether (50 °C, 16 h) and deprotected with TBAF (1 equiv.) in THF (rt, 12 h) furnishing the chiral diol 4 in 60 % yield for the two last steps. The overall yield (from 1) is 30% and the optical purity of the diol (*R*,*R*)-4 is 99 % *ee* (chiral GC analysis) after recrystallization in AcOEt (mp 72-73 °C, [α]²⁰_{D} -22.3 (*c* 1.21, CHCl₃)).

### Example 2 Preparation of (1S,2S)/(1R,2R) trans-cyclohex-3-en-1,2-diol using enzymatic reaction

40 mmol (7.92 g) of racemic 3,4 trans-diacetoxy-cyclohexen is resolved at 38°C in 1600 ml 0.075M phosphate buffer (pH 7) using 30,970U (10.0 mg) *Pseudomas Fluorescens Lipase* (Fluka). The progress of the reaction is followed and the reaction is stopped, when the *ee* of the unsaturated diacetate reaches 92% (22hr). After saturation with NaCl, extraction with 4 portions of 500ml diethyl ether, drying of the combined extracts with MgSO₄ and evaporation of the solvent the residue is chromatographied on silica to give 3.56 g (45% yield) of the unreacted diacetate (S,S 98% *ee)* and 2.92 g (47%) of the monoacetylated (3:4 mixture of isomers) product (R,R 95% *ee).*

17.5 mmol (3.5 g) of (1S,2S)-cyclohex-3-en-1,2-diacetate was dissolved in 30 ml dry methanol and treated with a catalytic amount of sodium methoxide. After standing 1 hour at room temperature TLC analysis indicated the complete hydrolysis of the diacetate. Removal of the solvent in vacuum, followed by distillation (140°C, oil pump) afforded 1.94 g (97%) of (S,S) trans-cyclohex-3-en-1,2-diol as a white solid.

18.5 mmol (2.90 g) of a 3:4 mixture of the monoacetylated derivatives of (R,R) trans-cyclohex-3-en-1,2-diol is dissolved in 30 ml dry methanol and treated with 2ml 10M sodium hydroxide (20 mmol). After standing over night TLC analysis indicated the complete hydrolysis of the monoacetate. Neutralization by the subsequent addition of 3 ml 2M HCl and excess NaHCO₃ is followed by the removal of the solvent in vacuum. Extraction with 3 portions of 50 ml dichloromethane followed by drying over MgSO₄ and removal of the solvent in vacuum afforded 1.49 g (71%) of (R,R) trans-cyclohex-3-en-1,2-diol as a white solvent.

### Example 3-5 Preparation of chiral 1,2-trans-bis(dihydrocarbyl phosphino oxide)-cyclohex-3-ens

The reaction steps are monitored by ³¹P NMR of an aliquot until completion of the reactions. A 250-mL-flask under argon equipped with a magnetic stirrer is charged with (1R,2R) trans-cyclohex-3-en-1,2-diol (10 mmol, 1.14 g), DMAP (21 mmol, 2.1 equiv., 2.57 g) and ether (120 mL). To the homogeneous solution, pure chlorodihydrocarbylphosphine (2.05 equiv.) is added dropwise. The resulting suspension is stirred for 0.5 h and then filtrated under argon over a short pad of dry silicagel. The precipitate is washed twice with ether (20 mL). The etheral solution is evaporated under reduced pressure and the resulting crude diphosphinite is diluted in toluene (80 mL) (for Example 3, 4) or in mesitylene (60 mL) (for Example 5). This solution is heated to reflux for 42 hours (for Example 3 and 4) or to 170 °C (oil bath) for 17 h (for Example 5). After cooling to rt, solvent is evaporated. The crude product of example 3 is directly recrystallized from AcOEt yielding a colorless crystallin solid (4.09 g, 85 % yield, mp 201-202 °C, [α]²⁰_{D}-68.7 (*c* 1.23, CHCl₃)). Crude product of example 4 is purified by chromatography (pentane-ether-CH₂Cl₂-methanol 1:0.5:0.5:0 to 0:1:1:0.1) followed by recrystallization in heptane/chloroform yielding white needles (complexe 1:1 with chloroform) (4.50 g, 63 % yield, mp 155-160 °C, [α]²⁰_{D} -70.4 (c 1.1, CHCl₃)). Crude product of example 5 is purified by chromatography (ether-CH₂Cl₂-methanol 1:1:0 to 1:1:0.1) followed by trituration in pentane-ether yielding a white microcrystallin solid (3.61 g, 81 % yield, mp 104-108 °C, [α]²⁰_{D} -92.5 (*c*.1.07, CHCl₃)). The results of examples 3-5 are summarized in table 1.

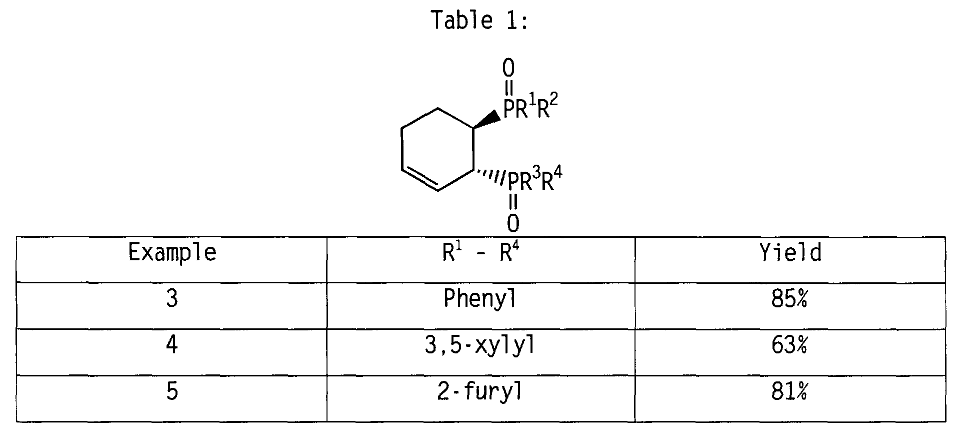

### Example 6-8 Preparation of chiral 1,2-trans-bis(dihydrocarbyl phosphino)-cyclohex-3-ens

The chiral phosphine oxide prepared in each of example 3-5 is charged in an autoclave under argon with toluene (30 mL) and trichlorosilane (4 mL, 40 mmol, 20 equiv. ) and heated for 14 h at 110 °C. After cooling to rt, the reaction mixture is transferred in a 100-mL-flask filled with argon. Toluene and trichlorosilane in excess are evaporated with high vacuum pump. The residue is dissolved in toluene (25 mL) and carefully hydrolyzed with degassed 3M aqueous KOH (15 mL). The mixture is stirred at 50 °C until the organic and aqueous layers become clear. Then the two layers are separated and the organic phase is dried (MgSO₄) under argon. The resulting clear and slightly yellow organic phase is filtered and transferred by cannulation in a second flask flushed with argon. Toluene is evaporated and the crude diphosphine is recrystallized or simply washed with dried degassed methanol. After filtration, traces of solvent are pumped off under high vacuum for 2 h yielding diphosphines as white or slightly yellow microcrystallin solid which are stored under argon. The results of examples 3-5 are summarized in table 1.

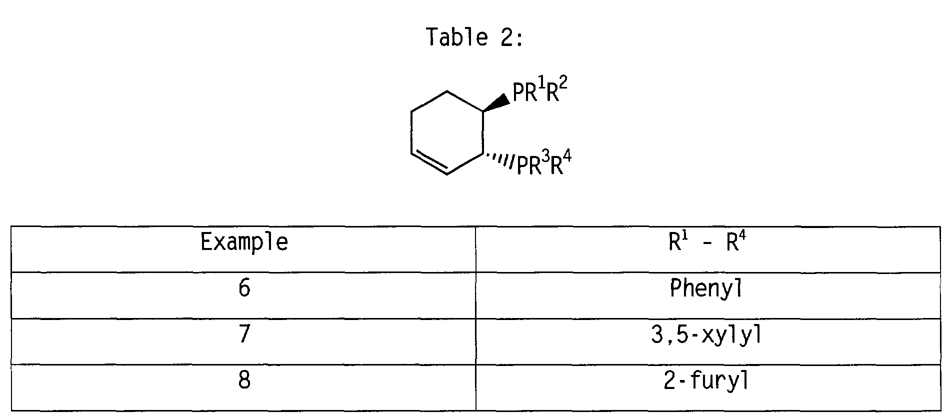

### Example 9-11 Preparation of chiral 1,2-trans-bis(dihydrocarbyl phosphino)cyclohexans

A 250-mL-flask under argon is charged with the unsaturated phosphine of each of examples 6-8 (2 mmol), wet 10 % palladium on charcoal (100 % in weight, from Degussa, type E 101 N/W 10 % containing 56 % water) and acetic acid (30 mL). The flask is purged for 15 min with H₂ under vigourous stirring and then heated to 65 °C for 18 h. After cooling to rt, Pd/C is filtrated and solvents are evaporated under reduced pressure. The residue is dissolved in ether-CH₂Cl₂ (1:1) and washed with K₂CO₃ solution, brine and dried over MgSO₄. After filtration, solvents are evaporated and the products recovered. The results of examples 9-11 are summarized in table 3.

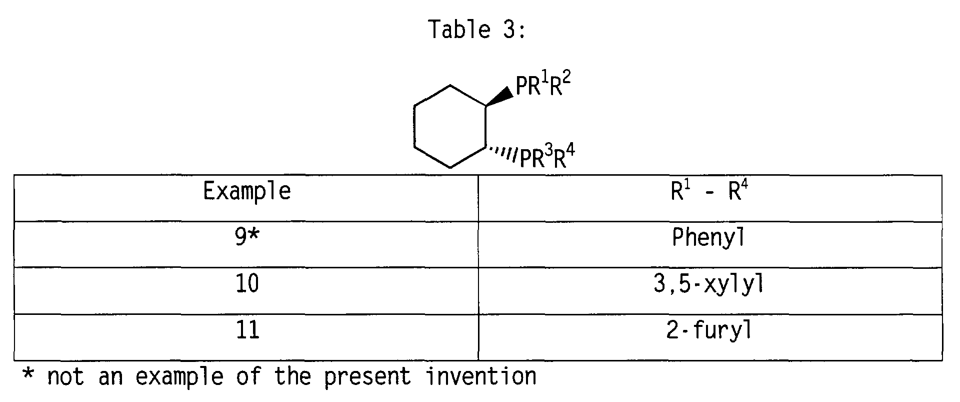

### Example 12 Preparation of (R,R)-1,2-bis(dicyclohexylphosphinyl) cyclohexan

The reaction product of example 3 is reduced by using Raney nickel as catalyst in accordance with the method disclosed in Morimoto, T. et al, Chem. Pharm. Bull., 1993, 41, 1149, leading to (R,R)-1,2-bis(dicyclohexylphosphineoxide)cyclohexan. The corresponding (R,R)-1,2-bis(dicyclohexylphosphinyl)cyclohexan is obtained in 90% yield following the procedure of Examples 6-8.

### Example 13 Preparation of chiral

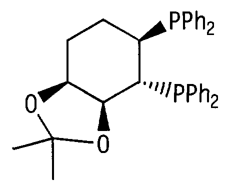

The product of example 6 is first protected with BH₃. Then the resulting protected diphosphine is treated with a catalytic amount of OsO₄ (10 mol %), NMO (3 equiv.) and pyridine (3 equiv.) in *tert*-BuOH/H₂O (reflux, 6h) The resulting stereoselectively obtained *cis*-1,2-diol is protected with 2,2-dimethoxypropane to form the acetal. Finally the deprotection step is ensured by treating the compound with a large excess of Et₂NH (100 equiv.) at 50°C overnight to yield the chiral diphosphine.

### Example 14 Hydroboration of styrene

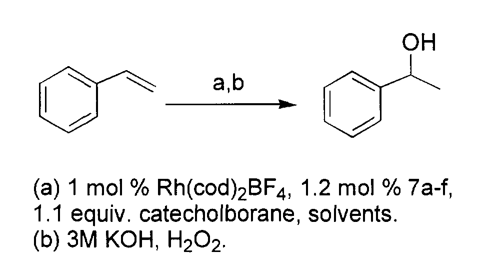

The hydroboration of styrene with catecholborane in the presence of Rh(COD)₂BF₄ (1 mol %) and the chiral diphosphines of examples 9-13 (1.2 mol %) furnishes after oxidative workup (KOH, H₂O₂) 1-phenylethanol with high regioselectivity (>99:1) and variable enantioselectivity. In a 10-mL-Schlenk-tube under argon, Rh(COD)₂BF₄ (8.1 mg, 0.02 mmol) and the respective diphosphine (11.5 mg, 0.024 mmol) in the solvent given in Table 4 (2 mL) are stirred for 0.5 h at rt. Styrene (2 mmol, 0.23 mL) is added dropwise to the resulting dark orange solution. The mixture is cooled to the temperature given in table 4 and stirred at this temperature for 15 min before adding dropwise freshly distilled catecholborane (2.2 mmol, 0.24 mL). Catecholborane slowly dissolves in DME and some gas evolves from the reaction mixture. The conversion is monitored by taking aliquots, reacting them with KOH (3M) and 60 % H₂O₂ and finally extracting them with ether. Samples are analyzed by GC with column Chiralsil DEX-CB, isotherm 100 °C. The enantioselectivity was determined by chiral GC, column Chiralsil DEX-CB, isotherm 100 °C.

**Table 4**

| Chiral diphosphine of example | Solvent | Temp.(°C) | Enantio-Selectivity(%) |
|---|---|---|---|
| 9* | Et₂O/CH₂Cl₂ | -60 | 8 (R) |
| 10 | Et₂O/CH₂Cl₂ | -60 | 65 (R) |
| 11 | Et₂O/CH₂Cl₂ | -60 | 65 (S) |
| 12 | DME | -35 | 92 (S) |
| 13 | DME | -40 | 15 (R) |

| | | | |
|---|---|---|---|
| * not an example of the present invention | | | |

### Example 15 Hydrogenation of Ethylbenzoylacetate

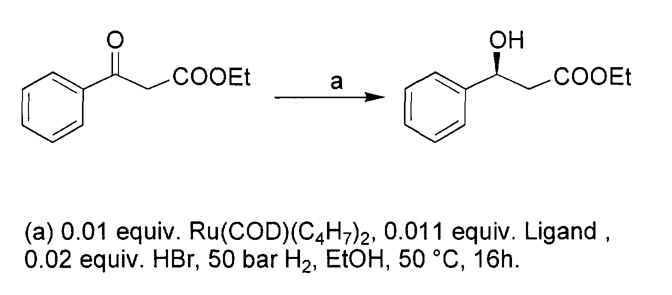

The ruthenium catalyst is prepared using the following procedure. The biphosphine ligand (0.011 mmol) and (COD) Ru (2-methylallyl)₂ (0.01 mmol) were placed in a Schlenk tube and purged with argon. Anhydrous acetone (2 ml) was added. To the resulting solution was added 0.08 mL (0.024 mmol) of a methanolic 0.3 M HBr solution (prepared from aqueous 48 % HBr solution). The resulting suspension was stirred at rt for 0.5 h. The mixture was evaporated under vacuum and the resulting catalyst was dissolved in 10 mL of ethanol. The solution of catalyst was transferred under argon in the autoclave containing 1 mmol of substrate and previously flushed by 3 cycles of vacuum/argon. The autoclave was purged with argon and then with hydrogen. The pressure was adjusted to 50 bar and the autoclave was heated to 50 °C, the pressure was maintained for 48 h. The reaction mixture was then concentrated to dryness. The residue was filtered on silicagel and eluted with Et₂O. The solvent was removed under vacuum and the product analyzed by ¹H NMR and chiral HPLC. The enantiomeric excess was determined on chiral HPLC, column OD, eluent n-heptane-isopropanol (95:5), flow 0.9 mL/min, detector 213nm. The results are given in table 5.

**Table 5:**

| Chiral diphosphine of example | Conversion (%) | Enantio Selectivity (%) |
|---|---|---|
| 9* | 88 | 65 (*S*) |
| 10 | 55 | 68 (*S*) |
| 6 | 52 | 68 (*S*) |
| 13 | 93 | 75 (*S*) |

| | | |
|---|---|---|
| * not an example of the present invention | | |

### Example 16 Reduction of Imines

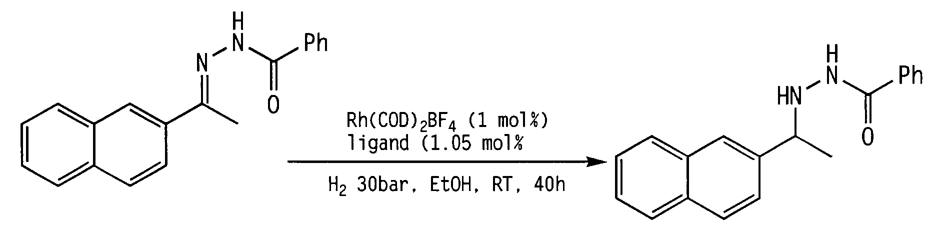

The rhodium catalyst is prepared using the following procedure. The diphosphine ligand (0,011 mmol) and Rh(COD)₂BF₄ (0.01 mmol) are placed in a Schlenk tube and purged with argon. Anhydrous ethanol (10ml) is added. The resulting solution is then transferred under argon in the autoclave containing 1 mmol of substrate and that is previously flushed with three cycles of vacuum/argon. The autoclave is purged with argon and then with hydrogen. The pressure is adjusted to 30 bars and is maintained for 40 hours. Then the reaction mixture is concentrated to dryness. The residue is filtered on silica gel and eluted with diethylether. The solvent is removed under vacuum. The enantiomeric excess was determined by chiral HPLC, column OJ, 40°C, heptane/isopropanol (92/8), flow 1 mL/min, detector 213nm. The results are given in table 6.

**Table 6:**

| Chiral diphosphine of example | Conversion (%) | Enantio Selectivity (%) |
|---|---|---|
| 10 | 61 | 23 |
| 6 | 100 | 61 |
| 13 | 49 | 36 |

The results of the asymmetric synthesis shown for examples 14-16 confirm that the chiral diphosphines of the present invention induce improved enantioselectivity especially if compared with chiral 1,2-trans-bis(diphenylphosphino) cyclohexan. This effect is pronounced for the asymmetric hydroboration (example 14). Furthermore the examples show that the present invention provides a versatile route to a variety of differently substituted chiral disphosphines so that a person skilled in the art is enabled to tailor a specific chiral diphosphine that gives the best result for a specific asymmetric synthesis.

## Claims

1. Chiral diphosphines selected from
a) diphosphines corresponding to the formula 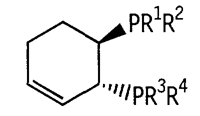 wherein R¹ - R⁴ are each independently selected from substituted and unsubstituted hydrocarbyl groups,
b) diphosphines corresponding to the formula 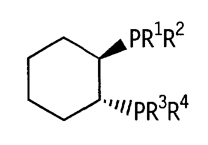 wherein R¹ - R⁴ are each independently selected from substituted and unsubstituted hydrocarbyl groups with the proviso that if R¹ and R² is phenyl at least one of R³ and R⁴ is different from phenyl
c) diphosphines corresponding to the formula 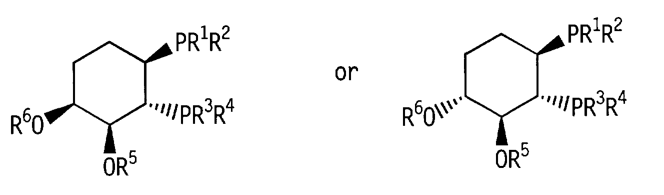
wherein R¹ - R⁴ are each independently selected from substituted and unsubstituted hydrocarbyl groups and R⁵ and R⁶ are each independently selected from the group consisting of hydrogen, alkyl and residues of protecting groups, whereby R⁶ and R⁵ taking together may be an divalent substituent thereby forming a bicyclus.

2. Chiral diphosphines of claim 1, wherein one of R¹ and R² is different from R³ and R⁴.

3. A method for preparing chiral diphosphines corresponding to the formula 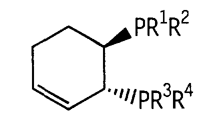 by reacting a chiral 1,2-diphosphine oxide corresponding to the formula 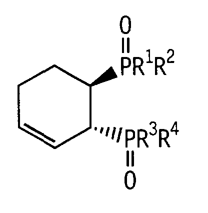 with an stoichiometric excess of hydrosilane wherein R¹ - R⁴ are each independently selected from substituted and unsubstituted hydrocarbyl groups.

4. The method of claim 3 for preparing the chiral diphosphine having identical phosphino groups wherein the starting chiral 1,2-diphosphine oxide is prepared by first reacting the chiral trans cyclohex-3-en-1,2-diol with ClPR¹R² wherein R¹ and R² is defined as in claim 4 to obtain the corresponding biphosphinite and thereafter effecting a [2,3]-sigmatropic rearrangement by heating the biphosphinite to obtain the chiral 1,2-diphosphine oxide.

5. The method of claim 3 for preparing the chiral diphosphine having different phosphino groups wherein the starting chiral 1,2-diphosphine oxide is prepared by
a) first reacting the chiral trans cyclohex-3-en-1,2-diol with a compound to protect one hydroxy functionality,
b) reacting product of step a) with ClPR¹R²,
c) deprotecting the protected hydroxy functionality of the product of step b),
d) reacting the resulting hydroxy functional compound of step c) with ClPR³R⁴ and
e) effecting a [2,3]-sigmatropic rearrangement by heating the product of step d) to obtain the chiral 1,2-diphosphine oxide.
whereby R¹ - R⁴ are each independently selected from substituted and unsubstituted hydrocarbyl groups, with the proviso that at least one of R¹ and R² is different from R³ and R⁴.

6. The method of claim 3 for preparing the chiral diphosphine having different phosphino groups wherein the starting chiral 1,2-diphosphine oxide is prepared by
a) reacting the chiral trans cyclohex-3-en-1,2-diol with a compound to protect one hydroxy functionality,
b) reacting the product of step a) with,
c) effecting a [2,3]-sigmatropic rearrangement by heating the product of step c),
d) removing the protecting group of the product of step c),
e) reacting the hydroxy functionality of the product of step d) with ClPR³R⁴,
f) effecting a [2,3]-sigmatropic rearrangement by heating the product of step e) to yield the chiral 1,2-diphosphine oxide having different diphosphino oxide groups,
whereby R¹ - R⁴ are each independently selected from substituted and unsubstituted hydrocarbyl groups, with the proviso that at least one of R¹ and R² is different from R³ and R⁴.

7. A method for preparing the chiral (1R,2R) trans cyclohex-3-en-1,2-diol suitable as starting compound for the method of any of claims 4 - 6 by the sequence of following steps:
1) reduction of 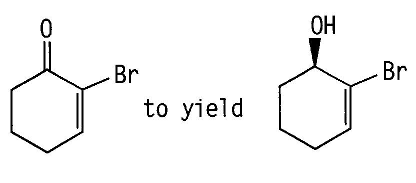
2) debromination and protection of the hydroxy functionality of 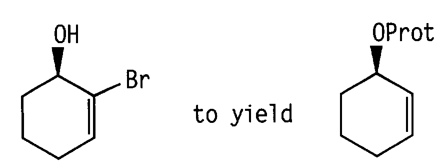 wherein Prot is a protecting group,
3) epoxidation of 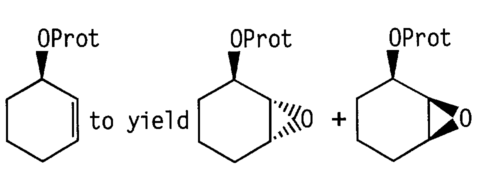
4) seperation of 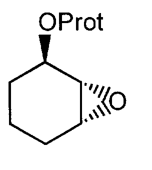
5) rearrangement of 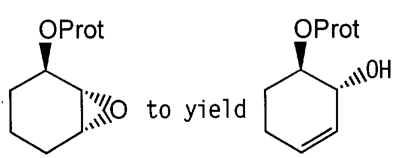
6) deprotection of

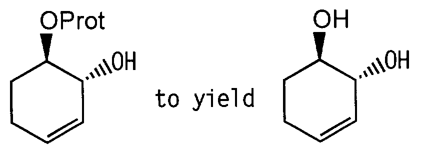

8. A method for preparing the chiral trans-cyclohex-3-en-1,2-diol suitable as starting compound for the method of any of claims 4 to 6 by the sequence of following steps:
a) reacting a racemic 3,4-trans-diacyloxy-cyclohexen in presence of an enzyme selective to convert one of the enantiomers of the racemate enantioselctively into chiral 3,4-trans-hydroxy-acyloxy-cyclohexen,
b) separating the unreacted enantiomer from the product of step a),
c) converting both fractions of step b) into the respective chiral trans-cyclohex-3-en-1,2-diol.

9. The method of claim 8, wherein the enzyme is *Pseudomas Fluorescens Lipase*.

10. A method for preparing chiral diphosphines corresponding to the formula a 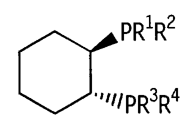 by hydrogenation of 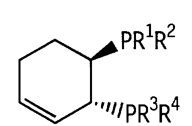 wherein R¹ - R⁴ are each independently selected from substituted and unsubstituted hydrocarbyl groups.

11. A method for preparing chiral diphosphines corresponding to the formula 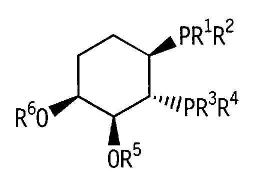 by cis-hydroxylation of 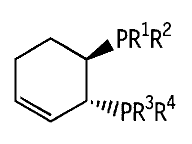 and optional subsequent alkylation or protection of the resulting hydroxy functionalities wherein R¹ - R⁴ are each independently selected from substituted and unsubstituted hydrocarbyl groups and R⁵ and R⁶ are each independently selected from the group consisting of hydrogen, alkyl and residues protecting groups, whereby R⁶ and R⁵ taking together may be an divalent substituent thereby forming a bicyclus.

12. A method for preparing chiral diphosphines corresponding to the formula 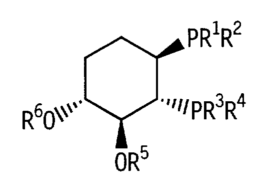 by trans-hydroxylation of 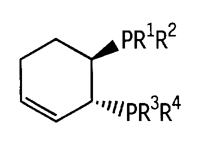 and optional subsequent alkylation or protection of the resulting hydroxy functionalities wherein R¹ - R⁴ are each independently selected from substituted and unsubstituted hydrocarbyl groups and R⁵ and R⁶ are each independently selected from the group consisting of hydrogen, alkyl and residues of protecting groups, whereby R⁶ and R⁵ taking together may be an divalent substituent thereby forming a bicyclus.

13. The method of any of claims 10-12, wherein the phosphino groups of 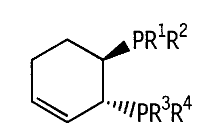 are protected by reaction with a borane prior to any subsequent reactions.

14. A transition metal complex comprising as ligand a chiral diphosphine according to any of claims 1 and 2.

15. The transition metal complex of claim 14, comprising a metal selected from metals of the groups 8, 9 and 10 of the periodic table of the elements.

16. Use of chiral diphosphines according to any of claims 1 and 2 or transition metal complexes according to any of claims 14 and 15 in enantioselective synthesis.

17. Use of claim 16 wherein the enantioselective reaction for synthesis is selected from hyrogenation and hydroboration of prochiral centers comprising unsaturated functionality in organic compounds.
